# EUROPEAN PATENT APPLICATION

(11) **EP 4 403 876 A1**
(43) Date of publication of application: **24.07.2024**
(21) Application number: 24151671.5
(22) Date of filing: 12.01.2024
(51) Int. Cl.: G01C 21/12, A61B 5/11, G01C 21/20, G01C 22/00

(54) **MEASUREMENT DEVICE, MEASUREMENT METHOD, AND PROGRAM**

(30) Priority: 19.01.2023 JP 2023006827
(71) Applicant: Casio Computer Co., Ltd., Tokyo 151-8543 (JP)
(72) Inventor: MATSUMOTO, Naoya, Tokyo, 205-8555 (JP)
(74) Representative: Plougmann Vingtoft a/s

(57) **Abstract**

A measurement device (100) includes an acceleration sensor (16), a memory (12), and one or more processors (11) that follow instructions saved in the memory (12) to execute following processing of: acquiring time-series acceleration data output by the acceleration sensor (16); specifying, based on the acquired time-series acceleration data, first and second timing at which acceleration goes above and below an acceleration reference value; determining a reference range for a time width from the first to second timing based on amplitude of an acceleration waveform corresponding to the time-series acceleration data and a first piece count of at least either the first or second timing within predetermined time; determining whether the time width falls within the determined reference range; and counting one step for a partial waveform from the first to second timing in the acceleration waveform when the time width is determined as falling within the reference range.

## Description

The present disclosure relates to a measurement device, a measurement method, and a program.

Currently, there is known a measurement device that measures the number of steps taken by a user walking or running based on an output of an acceleration sensor attached to the user. In such a measurement device, various threshold values are preferably determined according to a user's state. For example, Unexamined Japanese Patent Application Publication No. 2020-101849 describes a technique for determining a threshold value relating to a time width for one step in an acceleration waveform according to a user's state estimated from amplitude of the acceleration waveform.

In the technique described in Unexamined Japanese Patent Application Publication No. 2020-101849, the more the user's state is a state where the amplitude of the acceleration waveform increases, the shorter the time width for one step in the acceleration waveform is assumed to be. Specifically, when the user's state is a running state, the amplitude of the acceleration waveform is assumed to be large and the time width for one step in the acceleration waveform is assumed to be short. Further, when the user's state is a walking state, the amplitude of the acceleration waveform is assumed to be small and the time width for one step in the acceleration waveform is assumed to be long.

However, depending on the user's state, a relationship between the amplitude of the acceleration waveform and the time width for one step in the acceleration waveform may not be in the above-described relationship. For example, when the user's state is a state of running down stairs, the amplitude of the acceleration waveform may be small and the time width for one step in the acceleration waveform may be short. Accordingly, the technique described in Patent Literature 1 is also considered to have room for improving precision in step measurement. Thus, a technique for improving precision in step measurement is desired.

The present disclosure is made in view of the above problem, and an objective of the present disclosure is to provide a measurement device, a measurement method, and a program that improve precision in step measurement.

A measurement device according to one aspect of the present disclosure includes an acceleration sensor, a memory, and one or more processors.

The one or more processors follow instructions saved in the memory to execute following processing of:
acquiring time-series acceleration data output by the acceleration sensor;
specifying, based on the acquired time-series acceleration data, first timing at which acceleration goes above an acceleration reference value and second timing at which the acceleration goes below the acceleration reference value;
determining a reference range for a time width from the first timing to the second timing based on amplitude of an acceleration waveform corresponding to the time-series acceleration data and a first piece count of at least either the first timing or the second timing within predetermined time;
determining whether the time width falls within the determined reference range; and
counting one step for a partial waveform from the first timing to the second timing in the acceleration waveform when the time width is determined as falling within the reference range.

Further features of the present invention will become apparent from the following description of exemplary embodiments (with reference to the attached drawings).

A more complete understanding of this application can be obtained when the following detailed description is considered in conjunction with the following drawings, in which:
FIG. 1 is a configuration diagram of a measurement device according to the embodiment;
FIG. 2 is a functional configuration diagram of the measurement device according to the embodiment;
FIG. 3 is a diagram illustrating a resultant acceleration waveform after filter processing;
FIG. 4 is a diagram illustrating a part of the resultant acceleration waveform after offset processing;
FIG. 5 is an explanatory diagram of a reference range for a time width of a partial waveform;
FIG. 6 is a diagram illustrating a correspondence relationship between the time width of the partial waveform and amplitude of the resultant acceleration waveform;
FIG. 7 is a diagram illustrating the amplitude of the resultant acceleration waveform, the time width of the partial waveform, and a lower-limit threshold value of the time width for each user's state;
FIG. 8 is a diagram illustrating a correspondence relationship between maximum acceleration and the lower-limit threshold value;
FIG. 9 is a diagram illustrating a correspondence relationship between a maximum difference value and an amplitude threshold value;
FIG. 10 is a flowchart illustrating step measurement processing executed by the measurement device according to the embodiment;
FIG. 11 is a flowchart illustrating reference range determination processing illustrated in FIG. 10; and
FIG. 12 is a diagram illustrating a measurement result of a measurement error.

Embodiments of the present disclosure are described below in detail with reference to drawings. Note that, the same or equivalent parts in the drawings are denoted by the same reference signs.

### Embodiment

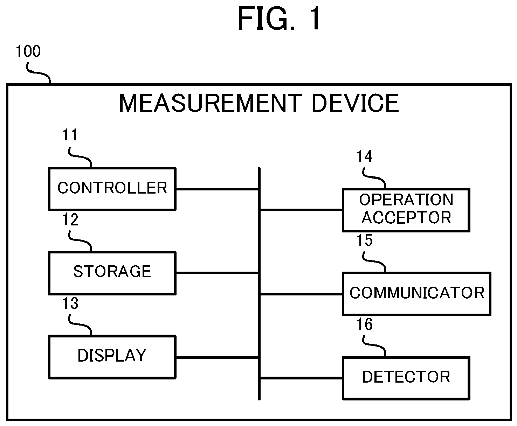

FIG. 1 is a diagram illustrating a configuration of a measurement device 100 according to Embodiment 1. The measurement device 100 is a device that measures the number of steps taken by a user walking or running. The measurement device 100 is worn on a user's body and measures the number of steps by detecting acceleration applied to the user's body. The measurement device 100 is worn on, for example, a user's arm, a user's hip, or the like. The measurement device 100 may be dedicated electronic equipment for measuring the number of steps, or may be general-purpose electronic equipment having a function of measuring the number of steps. The dedicated electronic equipment for measuring the number of steps is a so-called pedometer. Examples of the general-purpose electronic equipment include a smartphone, a smartwatch, and a mobile phone. As illustrated in FIG. 1, the measurement device 100 includes a controller 11, a storage 12, a display 13, an operation acceptor 14, a communicator 15, and a detector 16.

The controller 11 includes a central processing unit (CPU), a read-only memory (ROM), a random access memory (RAM), a real-time clock (RTC), and the like. The CPU is also called a processor, a microprocessor, a microcomputer, a digital signal processor (DSP), or the like, and functions as a central arithmetic processor that executes processing and operation relating to control of the measurement device 100. The CPU in the controller 11 reads out a program and data stored in the ROM, and integrally controls the measurement device 100 by using the RAM as a work area. The RTC is, for example, an integrated circuit having a clocking function. Note that, the CPU is capable of specifying a current date and time from time information read out from the RTC.

The storage 12 includes a non-volatile semiconductor memory such as a flash memory, an erasable programmable read-only memory (EPROM), or an electrically erasable programmable read-only memory (EEPROM), and serves as a so-called auxiliary storage device. The storage 12 stores a program and data used by the controller 11 to execute various kinds of processing. Further, the storage 12 stores data generated or acquired by the controller 11 executing various kinds of processing.

The display 13 displays various images according to control by the controller 11. For example, the display 13 displays a screen for accepting various operations from a user. The display 13 includes a touch screen, a liquid crystal display, and the like. The operation acceptor 14 accepts various operations from a user and supplies information indicating contents of the accepted operations to the controller 11. The operation acceptor 14 includes a touch screen, a button, a lever, and the like.

The communicator 15 communicates with various devices in accordance with a well-known wired communication standard or a well-known wireless communication standard, according to control by the controller 11. Examples of the well-known wired communication standard include Universal Serial Bus (USB, registered trademark) and Thunderbolt (registered trademark). Examples of the well-known wireless communication standard include Wi-Fi (registered trademark), Bluetooth (registered trademark), and Zigbee (registered trademark). The communicator 15 includes a communication interface complying with various communication standards.

The detector 16 detects acceleration applied to the measurement device 100. The detector 16 includes, for example, a three-axis acceleration sensor that detects acceleration in an X-axis direction, acceleration in a Y-axis direction perpendicular to the X-axis direction, and acceleration in a Z-axis direction perpendicular to the X-axis and Y-axis directions. The detector 16 outputs time-series acceleration data for each of the three axial directions. In other words, the detector 16 outputs time-series data indicating the acceleration in the X-axis direction, time-series data indicating the acceleration in the Y-axis direction, and time-series data indicating the acceleration in the Z-axis direction.

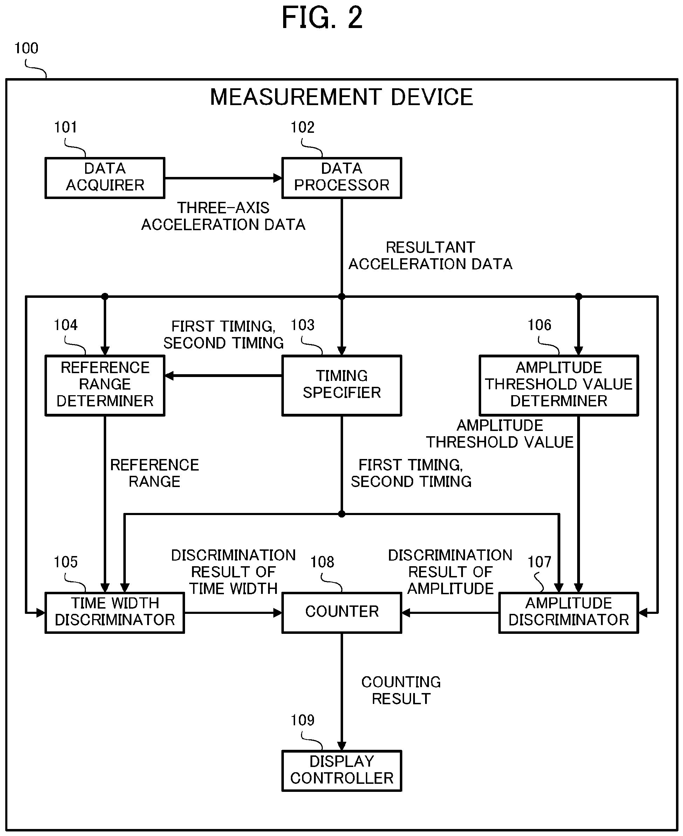

Next, functions of the measurement device 100 are described with reference to FIG. 2. The measurement device 100 functionally includes a data acquirer 101, a data processor 102, a timing specifier 103, a reference range determiner 104, a time width discriminator 105, an amplitude threshold value determiner 106, an amplitude discriminator 107, a counter 108, and a display controller 109. Each of the functions is achieved by software, firmware, or a combination of software and firmware. The software and the firmware are written as a program and stored in the ROM or the storage 12. Then, each of the functions is achieved by the CPU executing the program stored in the ROM or the storage 12.

The data acquirer 101 acquires time-series acceleration data output by an acceleration sensor. This acceleration sensor is, for example, the three-axis acceleration sensor included in the detector 16. The data acquirer 101 acquires time-series acceleration data on each axial direction from this three-axis acceleration sensor. The data acquirer 101 is one example of data acquisition means.

The data processor 102 performs various kinds of processing on the time-series acceleration data acquired by the data acquirer 101. For example, the data processor 102 executes combining processing, filter processing, offset processing, and the like. The combining processing is processing of combining pieces of the acceleration for the three axes. The combining processing is, for example, processing of calculating a square root of a sum of squares of the acceleration in the X-axis direction, the acceleration in the Y-axis direction, and the acceleration in the Z-axis direction for the acceleration at each time, thereby finding the calculated square root of the sum of squares as resultant acceleration. The combining processing calculates time-series resultant acceleration data.

The filter processing is processing of removing a high-frequency component from the time-series resultant acceleration data acquired by the combining processing. Generally, a limit of pitch during human walking or running is about 5 Hz. In view of this, in the filter processing, a signal component of 5 Hz or more is removed as a noise component. For example, when a sampling frequency of acceleration data is 32 Hz, a high-frequency component is removed by a low-pass filter with seven taps and tap coefficients of 1, 2, 3, 4, 3, 2, 1 from time-series resultant acceleration. The filter processing calculates high-frequency component-removed time-series resultant acceleration data.

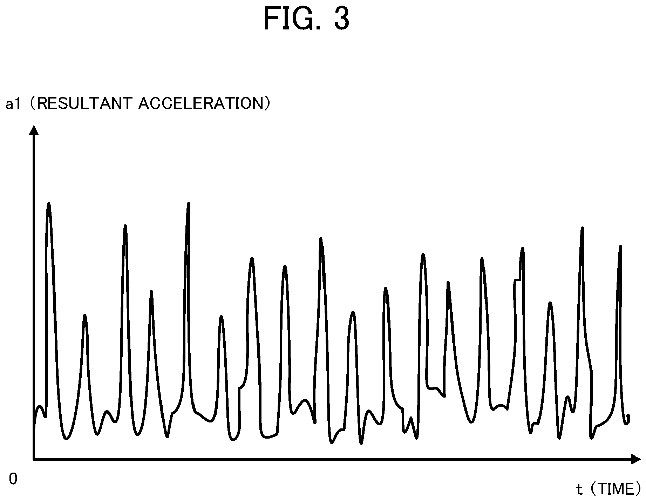

FIG. 3 illustrates a resultant acceleration waveform after the filter processing acquired during user running. The resultant acceleration waveform is a waveform corresponding to the time-series resultant acceleration data. In FIG. 3, a horizontal axis indicates time t and a vertical axis indicates resultant acceleration a1 after the filter processing. Note that, the sampling frequency of acceleration is 32 Hz in the present embodiment. When a user is running, the resultant acceleration basically fluctuates periodically, as illustrated in FIG. 3. Further, the resultant acceleration acquired when a user is walking also basically fluctuates periodically. Note that, amplitude of the resultant acceleration acquired during walking is basically smaller than amplitude of the resultant acceleration acquired during running. Further, a period of fluctuation of the resultant acceleration acquired during walking is basically longer than a period of fluctuation of the resultant acceleration acquired during running.

In FIG. 3, one peak of the resultant acceleration waveform can be basically regarded as one step. The present embodiment focuses on a partial waveform that is a waveform corresponding to one peak of the resultant acceleration waveform, and discriminates whether this partial waveform is a waveform originating from walking or running, based on both width and amplitude of the partial waveform. When this partial waveform is discriminated as a waveform originating from walking or running, one step is counted. The width of the partial waveform basically corresponds to pitch of walking or running. The amplitude of the partial waveform basically corresponds to magnitude of acceleration change caused by walking or running.

The offset processing is processing of removing a DC component from the time-series resultant acceleration data acquired by the filter processing. When finding the number of steps from acceleration change caused by walking or running, the DC component of acceleration is unnecessary for . In view of this, the offset processing removes the DC component as an offset component from the time-series resultant acceleration. For example, when the sampling frequency of acceleration data is 32 Hz, a DC component of the time-series resultant acceleration is calculated by an average filter with seventeen taps and tap coefficients of 1. Then, the calculated DC component is removed from each piece of the time-series resultant acceleration. The offset processing calculates DC component-removed time-series resultant acceleration data.

The timing specifier 103 specifies first timing and second timing based on the time-series acceleration data acquired by the data acquirer 101. The first timing is timing at which the acceleration goes above an acceleration reference value. The second timing is timing at which the acceleration goes below the acceleration reference value. The acceleration reference value is, for example, a value corresponding to the DC component of the acceleration indicated by the time-series acceleration data. Specifically, for example, the acceleration reference value is an average value of acceleration in recent prescribed time. The prescribed time is, for example, 1 second.

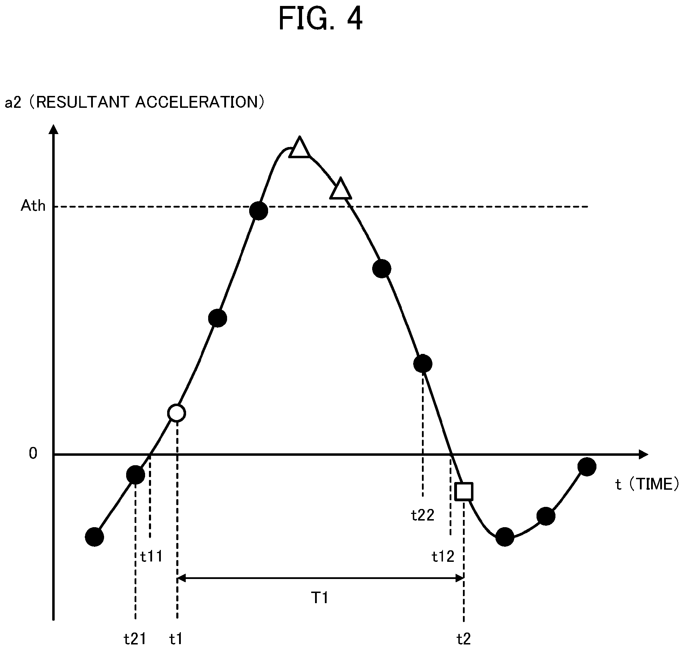

In the resultant acceleration waveform corresponding to the DC component-removed time-series resultant acceleration data, the first timing is timing of crossing zero toward a positive direction, and the second timing is timing of crossing zero toward a negative direction. For example, the timing specifier 103 specifies the first timing and the second timing based on the DC component-removed time-series resultant acceleration data calculated by the data processor 102. FIG. 4 illustrates a part of the resultant acceleration waveform corresponding to the DC component-removed time-series resultant acceleration data. In FIG. 4, a horizontal axis indicates time t and a vertical axis indicates resultant acceleration a2 after the offset processing.

In FIG. 4, the resultant acceleration during ascending crosses zero at timing 111, and the resultant acceleration during descending crosses zero at timing t12. Accordingly, t11 is preferably specified as the first timing, and 112 is preferably specified as the second timing. However, specifying t11 and t12 requires, for example, large processing load associated with interpolation processing. In view of this, in the present embodiment, timing t1 at a first point corresponding to the resultant acceleration that has changed from a negative value to a positive value among pieces of the time-series resultant acceleration is regarded as the first timing. Further, timing t2 at a second point corresponding to the resultant acceleration that has changed from a positive value to a negative value among pieces of the time-series resultant acceleration is regarded as the second timing.

Note that, the first timing at which the acceleration goes above the acceleration reference value is not limited to timing t1 immediately after a2 becomes zero. For example, the first timing may be timing t21 immediately before a2 becomes zero, may be timing 111 at which a2 becomes zero, or may be intermediate timing between timing t21 and timing t1 immediately before and after a2 becomes zero. Further, the second timing at which the acceleration goes below the acceleration reference value is not limited to timing t2 immediately after a2 becomes zero. For example, the second timing may be timing t22 immediately before a2 becomes zero, may be timing t12 at which a2 becomes zero, or may be intermediate timing between timing t22 and timing t2 immediately before and after a2 becomes zero.

In FIG. 4, the first point is indicated by a white circle, and the second point is indicated by a white square. In the present embodiment, a waveform from the first timing t1 to the second timing t2 of the DC component-removed resultant acceleration waveform is called a partial waveform. The higher the sampling frequency of the acceleration data, the smaller the difference between t11 and t1 and the difference between t12 and t2. Note that, discrimination as to whether the partial waveform is a waveform originating from walking or running is performed for all partial waveforms, as is described later. Then, a piece count of partial waveforms discriminated as a waveform originating from walking or running is set to the number of steps. The timing specifier 103 is one example of timing specification means.

The reference range determiner 104 determines a reference range for a time width from the first timing to the second timing based on amplitude of the acceleration waveform corresponding to the time-series acceleration data and a first piece count. The time width from the first timing to the second timing is a time width of one partial waveform. In other words, this second timing is second timing later than this first timing and adjacent to this first timing. Accordingly, the time width from the first timing to the second timing is a time width from specific first timing among a plurality of pieces of the first timing to second timing immediately after this specific first timing among a plurality of pieces of the second timing.

The first piece count is a piece count of at least either the first timing or the second timing within predetermined time. In other words, the first piece count may be a piece count of the first timing within the predetermined time, may be a piece count of the second timing within the predetermined time, or may be a sum of a piece count of the first timing within the predetermined time and a piece count of the second timing within the predetermined time. The predetermined time is, for example, recent 2 seconds.

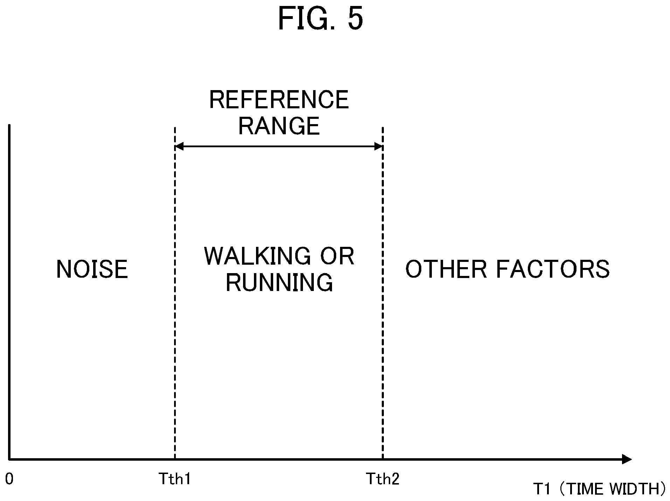

Here, one condition for the partial waveform to be discriminated as a waveform originating from walking or running is that a time width T1 of the partial waveform falls within the reference range. In other words, as illustrated in FIG. 5, when T1 falls within the reference range from Tth1 to Tth2, the partial waveform may be discriminated as a waveform originating from walking or running. Meanwhile, when T1 does not fall within the reference range from Tth1 to Tth2, the partial waveform is discriminated as not a waveform originating from walking or running.

Tth1 is a lower-limit value of the reference range, and is called a lower-limit threshold value as appropriate. Tth2 is an upper-limit value of the reference range, and is called an upper-limit threshold value as appropriate. When T1 is less than Tth1, the partial waveform is discriminated as a waveform originating from noise. Further, when T1 exceeds Tth2, the partial waveform is discriminated as a waveform originating from other factors. As described above, in the present embodiment, when the partial waveform included in the resultant acceleration waveform is a waveform originating from walking or running, the time width of this partial waveform is assumed to fall within the reference range.

Incidentally, the time width of the partial waveform is considered to vary depending on a user's state. For example, the time width of the partial waveform when a user is walking is typically larger than the time width of the partial waveform when the user is running. In view of this, the user's state is preferably estimated and the reference range for the time width of the partial waveform is preferably adjusted according to the user's state. In the present embodiment, the lower-limit threshold value being a lower-limit value of the reference range is adjusted according to the user's state, and the upper-limit threshold value being an upper-limit value of the reference range is a fixed value.

An approach to estimate the user's state can be adjusted as appropriate. For example, the user's state can be estimated from the amplitude of the acceleration waveform, more specifically, the amplitude of the DC component-removed resultant acceleration waveform. However, the user's state cannot easily be specified completely from only the amplitude of the resultant acceleration waveform. In view of this, in the present embodiment, the user's state is estimated in consideration of the above-described first piece count in addition to the amplitude of the resultant acceleration waveform.

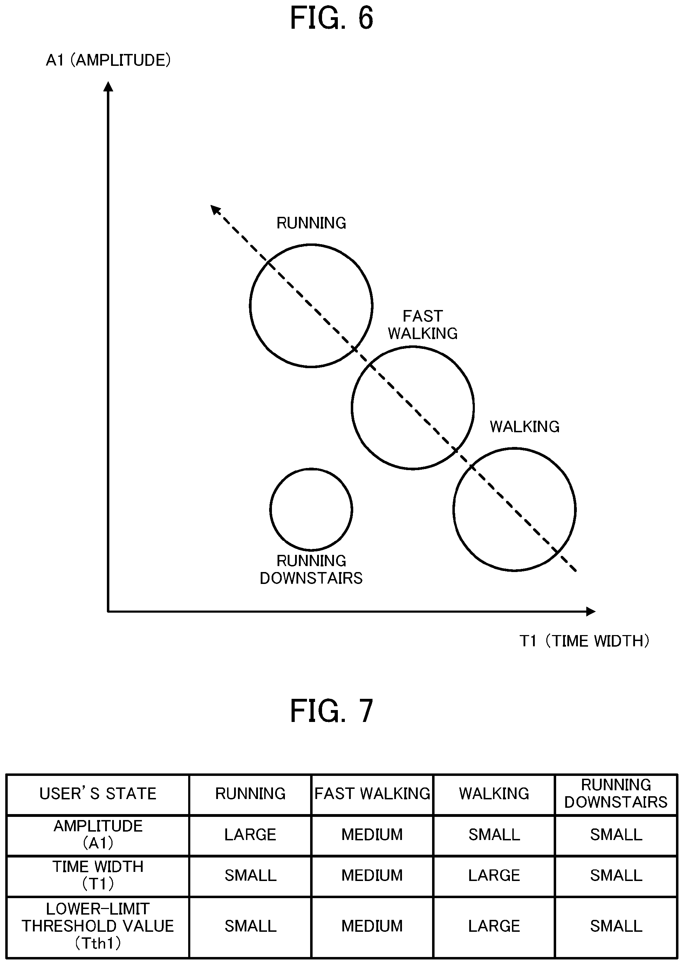

FIG. 6 illustrates a correspondence relationship among the time width of the partial waveform, the amplitude of the resultant acceleration waveform, and the user's state. In FIG. 6, a horizontal axis indicates the time width T1 of the partial waveform, and a vertical axis indicates the amplitude A1 of the resultant acceleration waveform. In FIG. 6, a running state, a fast walking state, a walking state, and a running downstairs state are indicated as the user's state. Note that, the running downstairs state is a state of running down stairs. FIG. 7 illustrates the amplitude of the resultant acceleration waveform, the time width of the partial waveform, and the lower-limit threshold value of the time width for each user's state.

As illustrated in FIG. 6, the time width of the partial waveform can be estimated from the user's state. Further, as illustrated in FIG. 6, when the running downstairs state is not taken into consideration, the user's state can be estimated from the amplitude of the resultant acceleration waveform. In other words, when the running downstairs state is not taken into consideration, the time width of the partial waveform can be estimated from the amplitude of the resultant acceleration waveform. In this case, the larger the amplitude of the resultant acceleration waveform, the smaller the time width of the partial waveform.

For example, when the amplitude of the resultant acceleration waveform is large, the user's state can be discriminated as the running state, and the time width of the partial waveform can be discriminated as small. Further, for example, when the amplitude of the resultant acceleration waveform is a medium level, the user's state can be discriminated as the fast walking state, and the time width of the partial waveform can be discriminated as a medium level. Further, for example, when the amplitude of the resultant acceleration waveform is small, the user's state can be discriminated as the walking state, and the time width of the partial waveform can be discriminated as large.

Meanwhile, when the running downstairs state is taken into consideration, the user's state is difficult to be estimated from the amplitude of the resultant acceleration waveform. In other words, when the running downstairs state is taken into consideration, the user's state is difficult to be estimated from the amplitude of the resultant acceleration waveform, and thus, the time width of the partial waveform is difficult to be estimated from the amplitude of the resultant acceleration waveform. For example, when the amplitude of the resultant acceleration waveform is small, the user's state is difficult to be discriminated as either the walking state or the running downstairs state, and the time width of the partial waveform is difficult to be estimated.

As described above, the user's state is difficult to be estimated completely only from the amplitude of the resultant acceleration waveform. In view of this, in the present embodiment, the user's state is estimated in consideration of the above-described first piece count in addition to the amplitude of the resultant acceleration waveform. In other words, the user's state is refined from the amplitude of the resultant acceleration waveform, by considering that the larger the fluctuation of acceleration caused by walking or running, the larger the amplitude of the resultant acceleration waveform. Further, the user's state is further refined from the first piece count, by considering that the faster the pitch of walking or running, the larger the first piece count.

For example, when the user's state is the walking state, the fluctuation of acceleration caused by walking is considered to be small, and the amplitude of the resultant acceleration waveform is considered to be small. Further, when the user's state is the running downstairs state, the fluctuation of acceleration originating from running downstairs is considered to be small, and the amplitude of the resultant acceleration waveform is considered to be small. Thus, the user's state is difficult to be discriminated as either the walking state or the running downstairs state only from the amplitude of the resultant acceleration waveform.

Here, when the user's state is the walking state, the pitch of walking is considered to be slow, and the first piece count is considered to be small. Further, when the user's state is the running downstairs state, the pitch of walking is considered to be fast, and the first piece count is considered to be large. Thus, the user's state can be discriminated as either the walking state or the running downstairs state from the first piece count. In other words, in the present embodiment, the user's state is estimated from the amplitude of the resultant acceleration waveform and the first piece count, and the time width of the partial waveform is estimated from the estimated user's state.

The reference range determiner 104 sets the lower-limit threshold value according to the estimated time width. Specifically, as illustrated in FIG. 7, the reference range determiner 104 increases the lower-limit threshold value the larger the estimated time width is. For example, when the user's state is estimated to be the running state, the reference range determiner 104 decreases the lower-limit threshold value to prevent the partial waveform originating from running from being regarded as the partial waveform originating from noise. Further, when the user's state is estimated to be the walking state, the reference range determiner 104 increases the lower-limit threshold value to prevent the partial waveform originating from noise from being regarded as the partial waveform originating from walking.

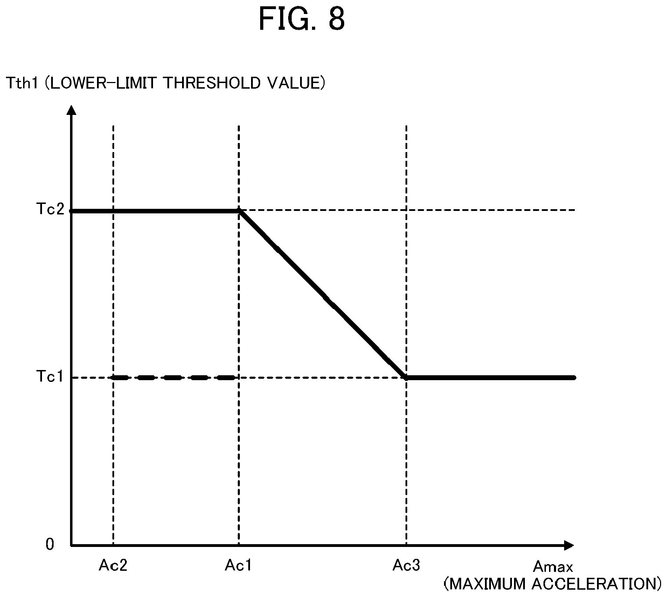

The lower-limit threshold value determined from the amplitude of the resultant acceleration waveform and the first piece count is described with reference to FIG. 8. An approach to find the amplitude of the resultant acceleration waveform can be adjusted as appropriate. In the present embodiment, maximum acceleration that is the maximum resultant acceleration in a recent 1 second among pieces of the DC component-removed time-series resultant acceleration is regarded as the amplitude of the resultant acceleration waveform. In other words, this maximum acceleration Amax is regarded as the amplitude A1 of the resultant acceleration waveform. In FIG. 8, a horizontal axis indicates the maximum acceleration Amax, and a vertical axis indicates the lower-limit threshold value Tth1. Further, in FIG. 8, a thick solid line indicates the lower-limit threshold value when the user's state is any of the running state, the fast walking state, and the walking state, and a thick dashed line indicates the lower-limit threshold value when the user's state is the running downstairs state.

When the maximum acceleration Amax is equal to or more than Ac3, the reference range determiner 104 estimates the user's state to be the running state, and sets the lower-limit threshold value Tth1 to Tc1. Tc1 is, for example, 63 msec. When Amax is less than Ac3 and exceeds Ac1, the reference range determiner 104 estimates the user's state to be the fast walking state, and sets Tth1 to any value from Tc1 to Tc2. Ac1 is smaller than Ac3. Tc2 is larger than Tc1, and is, for example, 125 msec. Note that, when the user's state is estimated to be the fast walking state, the reference range determiner 104 decreases Tth1 the larger Amax is.

When Amax is equal to or less than Ac1 and the first piece count is less than a piece count threshold value or when Amax is less than Ac2, the reference range determiner 104 estimates the user's state to be the walking state, and sets Tth1 to Tc2. Ac2 is smaller than Ac1. The piece count threshold value is set to such a value as to be capable of distinguishing between a case in which the pitch of walking or running is equal to or more than 3 Hz and a case in which this pitch is less than 3 Hz.

For example, when the first piece count is a piece count of the first timing in recent 2 seconds, the piece count threshold value is set to 6. For example, when the first piece count is a piece count of the first timing or the second timing in recent 2 seconds, the piece count threshold value is set to 12. When Amax is equal to or less than Ac1 and equal to or more than Ac2 and the first piece count is equal to or more than the piece count threshold value, the reference range determiner 104 estimates the user's state to be the running downstairs state, and sets Tth1 to Tc1.

As described above, the reference range determiner 104 estimates the user's state based on the amplitude of the acceleration waveform and the first piece count, and determines the reference range based on the estimated user's state. For example, when the amplitude of the acceleration waveform is less than a first reference value and is equal to or more than a second reference value smaller than the first reference value and the first piece count is equal to or more than the piece count threshold value, the reference range determiner 104 estimates the user's state to be the running downstairs state of running down stairs. Ac2 is one example of the second reference value. Further, when the amplitude of the acceleration waveform is less than the first reference value and the first piece count is less than the piece count threshold value or when the amplitude of the acceleration waveform is less than the second reference value, the reference range determiner 104 estimates the user's state to be the walking state.

When the user's state is estimated to be the running downstairs state, the reference range determiner 104 determines the lower-limit value of the reference range as a first lower-limit value. Tc1 is one example of the first lower-limit value. When the user's state is estimated to be the walking state, the reference range determiner 104 determines the lower-limit value of the reference range as a second lower-limit value larger than the first lower-limit value. Tc2 is one example of the second lower-limit value. The reference range determiner 104 is one example of reference range determination means.

The time width discriminator 105 discriminates whether the time width from the first timing to the second timing falls within the reference range determined by the reference range determiner 104. Specifically, the time width discriminator 105 discriminates whether the time width T1 falls within a range from the lower-limit threshold value Tth1 to the upper-limit threshold value Tth2. In other words, the time width discriminator 105 discriminates whether the pitch assumed from the partial waveform is the pitch originating from walking or running. The time width discriminator 105 is one example of time width discrimination means.

The amplitude threshold value determiner 106 determines an amplitude threshold value based on the amplitude of the acceleration waveform. The amplitude threshold value is a threshold value for the amplitude of the partial waveform. In the present embodiment, the amplitude threshold value is determined based on a maximum difference value Adif that is a difference between the maximum acceleration Amax and minimum acceleration Amin in recent 1 second among pieces of the DC component-removed time-series resultant acceleration. Note that, this maximum difference value Adif can be regarded as a value about twice as large as the amplitude A1 of the resultant acceleration waveform.

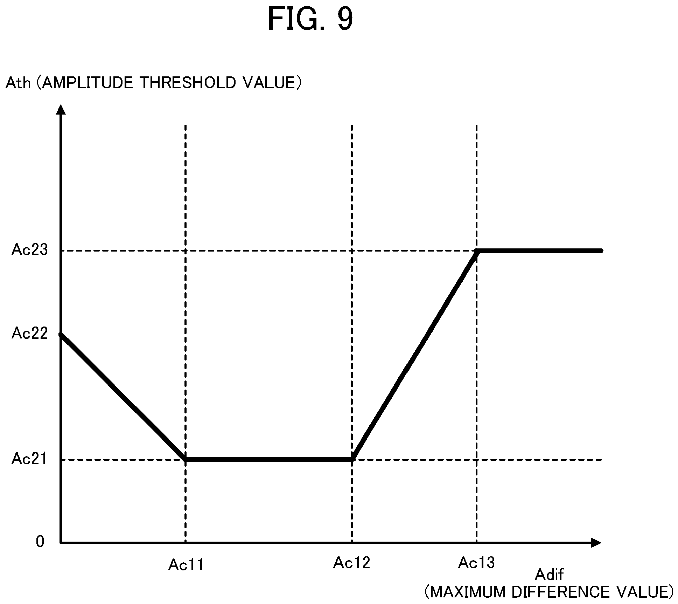

The amplitude threshold value determined from the maximum difference value is described with reference to FIG. 9. In FIG. 9, a horizontal axis indicates the maximum difference value Adif, and a vertical axis indicates the amplitude threshold value Ath. When the maximum difference value Adif is less than Ac11, the amplitude threshold value determiner 106 estimates that the user is less likely to be walking or running, and sets the amplitude threshold value Ath to any value from Ac21 to Ac22. Specifically, in this case, the amplitude threshold value determiner 106 increases Ath the smaller Adif is, in such a way as to prevent erroneous recognition of the number of steps. Ac22 is larger than Ac21.

When Adif is equal to or more than Ac11 and equal to or less than Ac12, the amplitude threshold value determiner 106 estimates that the user is highly likely to be walking or running with less fluctuation of acceleration, and sets Ath to Ac21. In other words, the amplitude threshold value determiner 106 sets Ath to a relatively small value Ac21 to reduce counting omission. The walking or running with less fluctuation of acceleration is, for example, non-fast walking, going down stairs, and the like. Note that, Ac12 is larger than Ac11.

When Adif is larger than Ac12 and less than Ac13, the amplitude threshold value determiner 106 estimates that the user is highly likely to be walking or running with a medium level of fluctuation of acceleration, and sets Ath to any value from Ac21 to Ac23. Specifically, the amplitude threshold value determiner 106 increases Ath the larger Adif is. Note that, even when Ath is increased the larger Adif is, counting omission is considered unlikely to occur. The walking or running with a medium level of fluctuation of acceleration is, for example, fast walking, jogging, and the like. Ac23 is larger than Ac22.

When Adif is equal to or more than Ac13, the amplitude threshold value determiner 106 estimates that the user is highly likely to be running, and sets Ath to Ac23. Note that, in this case, since Adif is sufficiently large, counting omission is considered unlikely to occur even when Ath is increased. As described above, the amplitude threshold value determiner 106 determines the amplitude threshold value based on the amplitude of the acceleration waveform. The amplitude threshold value determiner 106 is one example of amplitude threshold value determination means.

The amplitude discriminator 107 discriminates whether the amplitude of the partial waveform is equal to or more than the amplitude threshold value. For example, the amplitude discriminator 107 discriminates whether the resultant acceleration being equal to or more than the amplitude threshold value is included in pieces of the DC component-removed time-series resultant acceleration representing the partial waveform. For example, in FIG. 4, pieces of the resultant acceleration corresponding to two points indicated by white triangles are equal to or more than the amplitude threshold value Ath. In this case, the amplitude discriminator 107 discriminates that the amplitude of the partial waveform is equal to or more than the amplitude threshold value. The amplitude discriminator 107 is one example of amplitude discrimination means.

The counter 108 counts one step for the partial waveform when the time width discriminator 105 discriminates that the time width falls within the reference range and the amplitude discriminator 107 discriminates that the amplitude of the partial waveform is equal to or more than the amplitude threshold value. In other words, when the time width of the partial waveform is discriminated as the time width corresponding to the pitch of walking or running and the amplitude of the partial waveform is discriminated as the amplitude corresponding to the fluctuation amount of acceleration associated with walking or running, the counter 108 regards the partial waveform as a waveform originating from walking or running, and counts up the number of steps. The counter 108 is one example of counting means.

The display controller 109 displays a result of counting by the counter 108. For example, the display controller 109 controls the display 13 in such a way as to display the number of steps found by the counter 108.

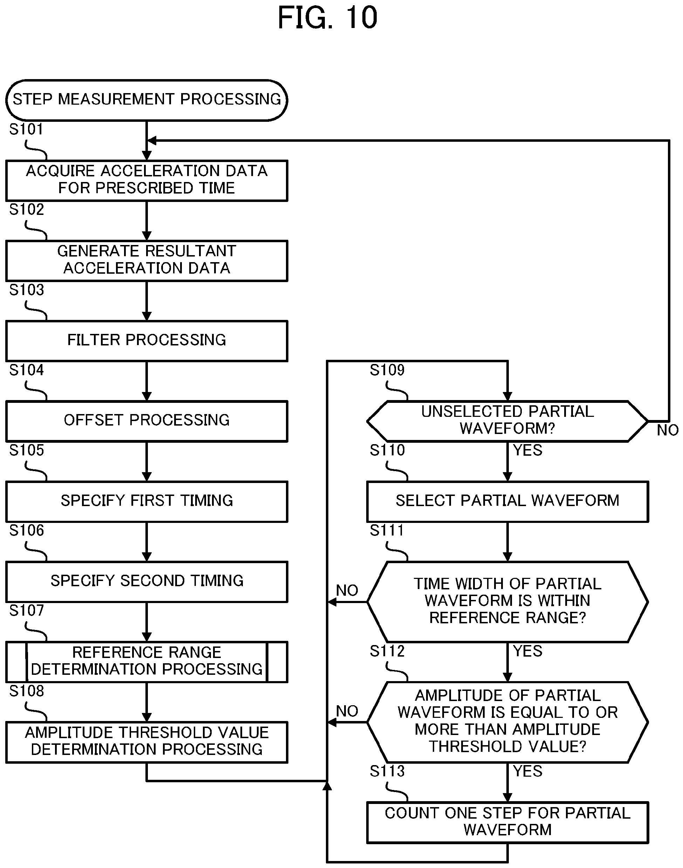

Next, step measurement processing executed by the measurement device 100 is described with reference to a flowchart in FIG. 10. The step measurement processing is executed, for example, in response to the measurement device 100 accepting an instruction from a user to start the step measurement processing.

First, the controller 11 included in the measurement device 100 acquires acceleration data for prescribed time (Step S101). For example, the controller 11 acquires, from the detector 16, acceleration data of the X-axis direction for 1 second, acceleration data of the Y-axis direction for 1 second, and acceleration data of the Z-axis direction for 1 second. After completing the processing in Step S101, the controller 11 generates resultant acceleration data (Step S102). For example, the controller 11 calculates resultant acceleration data for 1 second by finding a square root of the sum of squares of the acceleration data for the three directions at each time.

After completing the processing in Step S102, the controller 11 executes the filter processing (Step S103). In other words, the controller 11 applies a low-pass filter to the resultant acceleration data for 1 second to acquire high-frequency component-removed resultant acceleration data for 1 second. After completing the processing in Step S103, the controller 11 executes the offset processing (Step S104). In other words, the controller 11 finds an average value of pieces of the high-frequency component-removed resultant acceleration for 1 second, removes the average value from each piece of the high-frequency component-removed resultant acceleration for 1 second, and thereby acquires DC component-removed resultant acceleration data for 1 second.

After completing the processing in Step S104, the controller 11 specifies the first timing (Step S105). For example, the controller 11 specifies all points where the value goes from negative to positive in a resultant acceleration waveform indicated by the DC component-removed resultant acceleration data for 1 second. After completing the processing in Step S105, the controller 11 specifies the second timing (Step S106). For example, the controller 11 specifies all points where the value goes from positive to negative in a resultant acceleration waveform indicated by the DC component-removed resultant acceleration data for 1 second.

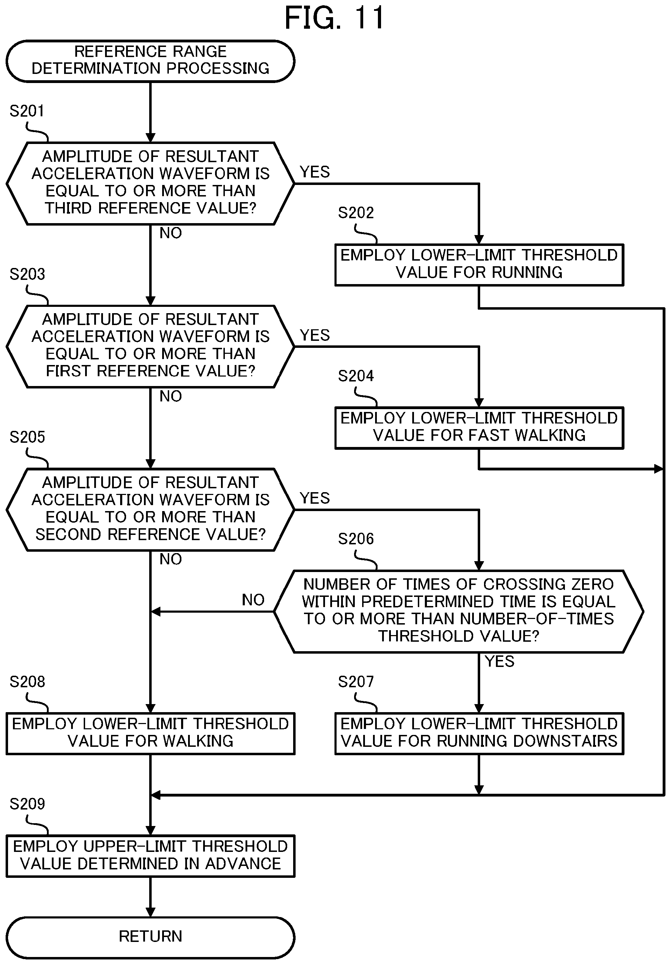

After completing the processing in Step S106, the controller 11 executes reference range determination processing (Step S107). The reference range determination processing is described in detail with reference to a flowchart illustrated in FIG. 11.

First, the controller 11 discriminates whether the amplitude of the resultant acceleration waveform is equal to or more than a third reference value (Step S201). For example, the controller 11 discriminates whether Amax is equal to or more than Ac3 in FIG. 8. When discriminating that the amplitude of the resultant acceleration waveform is equal to or more than the third reference value (Step S201: YES), the controller 11 employs a lower-limit threshold value for running (Step S202). For example, the controller 11 employs Tc1 as a lower-limit value of the reference range in FIG. 8.

When discriminating that the amplitude of the resultant acceleration waveform is not equal to or more than the third reference value (Step S201: NO), the controller 11 discriminates whether the amplitude of the resultant acceleration waveform is equal to or more than a first reference value (Step S203). For example, the controller 11 discriminates whether Amax is equal to or more than Ac1 in FIG. 8. When discriminating that the amplitude of the resultant acceleration waveform is equal to or more than the first reference value (Step S203: YES), the controller 11 employs a lower-limit threshold value for fast walking (Step S204). For example, the controller 11 employs any value from Tc1 to Tc2 as a lower-limit value of the reference range in FIG. 8.

When discriminating that the amplitude of the resultant acceleration waveform is not equal to or more than the first reference value (Step S203: NO), the controller 11 discriminates whether the amplitude of the resultant acceleration waveform is equal to or more than a second reference value (Step S205). For example, the controller 11 discriminates whether Amax is equal to or more than Ac2 in FIG. 8. When discriminating that the amplitude of the resultant acceleration waveform is equal to or more than the second reference value (Step S205: YES), the controller 11 discriminates whether the number of times of crossing zero within predetermined time is equal to or more than a number-of-times threshold value (Step S206). The number of times of crossing zero within predetermined time corresponds to the above-described first piece count. The number-of-times threshold value corresponds to the above-described piece count threshold value.

When discriminating that the number of times of crossing zero within predetermined time is equal to or more than the number-of-times threshold value (Step S206: YES), the controller 11 employs a lower-limit threshold value for running downstairs (Step S207). For example, the controller 11 employs Tc1 as a lower-limit value of the reference range in FIG. 8. When discriminating that the amplitude of the resultant acceleration waveform is not equal to or more than the second reference value (Step S205: NO) or when discriminating that the number of times of crossing zero within predetermined time is not equal to or more than the number-of-times threshold value (Step S206: NO), the controller 11 employs a lower-limit threshold value for walking (Step S208). For example, the controller 11 employs Tc2 as a lower-limit value of the reference range in FIG. 8.

After completing the processing in Step S202, Step S204, Step S207, or Step S208, the controller 11 employs an upper-limit threshold value determined in advance (Step S209). After completing the processing in Step S209, the controller 11 completes the reference range determination processing.

After completing the reference range determination processing in Step S107, the controller 11 executes amplitude threshold value determination processing (Step S108). For example, the controller 11 determines an amplitude threshold value from a maximum difference value, as described with reference to FIG. 9. After completing the processing in Step S108, the controller 11 discriminates whether there is an unselected partial waveform (Step S109). In other words, the controller 11 discriminates whether there is any partial waveform that is not selected in Step S110 among partial waveforms included in the resultant acceleration waveform for 1 second. Note that, the partial waveform is a waveform from the first timing to the second timing in the resultant acceleration waveform for 1 second. One or more partial waveforms may be included in the resultant acceleration waveform for 1 second, or no partial waveform may be included.

When discriminating that there is no unselected partial waveform (Step S109: NO), the controller 11 returns the processing to Step S101. Here, when the first timing is included in timing later than the latest second timing in the resultant acceleration waveform for 1 second, the resultant acceleration data after this latest second timing is carried over to subsequent processing. Such a configuration prevents detecting omission of the partial waveform, and prevents counting omission.

When discriminating that there is an unselected partial waveform (Step S109: YES), the controller 11 selects the partial waveform (Step S110). For example, the controller 11 selects the unselected partial waveform from among one or more partial waveforms included in the resultant acceleration waveform for 1 second. Note that, selecting the partial waveform corresponds to selecting a pair of the first timing and the second timing following after this first timing.

After completing the processing in Step S110, the controller 11 discriminates whether the time width of the partial waveform is within a reference range (Step S111). For example, the controller 11 discriminates whether T1 is within a range from Tth1 to Tth2 in the example illustrated in FIG. 5. When discriminating that the time width of the partial waveform is within the reference range (Step S111: YES), the controller 11 discriminates whether the amplitude of the partial waveform is equal to or more than the amplitude threshold value (Step S112). For example, the controller 11 discriminates whether there is any resultant acceleration exceeding Ath among pieces of the time-series resultant acceleration forming the partial waveform in the example illustrated in FIG. 4.

When discriminating that the amplitude of the partial waveform is equal to or more than the amplitude threshold value (Step S112: YES), the controller 11 counts one step for the partial waveform (Step S113). When discriminating that the time width of the partial waveform is not within the reference range (Step S111: NO), when discriminating that the amplitude of the partial waveform is not equal to or more than the amplitude threshold value (Step S112: NO), or when completing the processing in Step S113, the controller 11 returns the processing to Step S109.

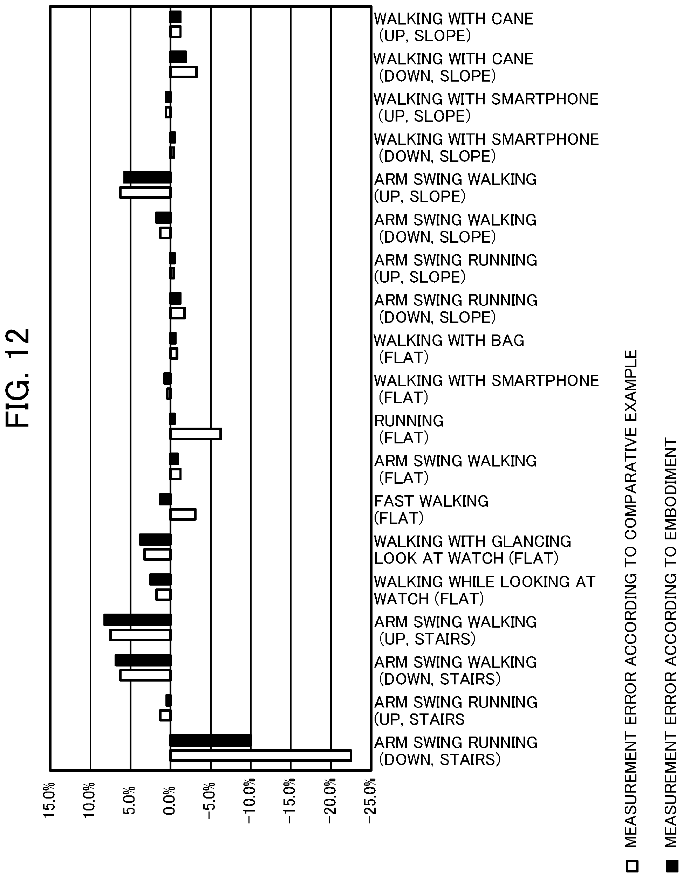

Next, an error in step measurement performed by the measurement device 100 is described with reference to FIG. 12. FIG. 12 illustrates measurement errors when step measurement is performed for various walking or running styles by using each of the measurement device 100 and a measurement device according to a comparative example. The measurement error in step measurement is represented as a percentage of (M-T)/T, where M is the number of steps acquired by step measurement and T is the true number of steps.

In FIG. 12, the measurement error by the measurement device according to the comparative example is indicated by a white square, and the measurement error by the measurement device 100 is indicated by a black square. Note that, the measurement device 100 performs step measurement in consideration of the first piece count, while the measurement device according to the comparative example performs step measurement without consideration of the first piece count. In other words, the measurement device 100 estimates the user's state from the amplitude of the resultant acceleration waveform and the first piece count, and determines a reference range for the time width of the partial waveform from the user's state. Meanwhile, the measurement device according to the comparative example estimates the user's state from the amplitude of the resultant acceleration waveform, and determines a reference range for the time width of the partial waveform from the user's state.

When the measurement error by the measurement device 100 is compared with the measurement error by the measurement device according to the comparative example, a large difference is observed in the case of going down stairs with arm swing running, while no large difference is observed in other walking or running styles. Specifically, in the case of going down stairs with arm swing running, the measurement error by the measurement device 100 is -9.9 ± 13.0%, while the measurement error by the measurement device according to the comparative example is -22.5 ± 19.5%. Further, the overall measurement error by the measurement device 100 is 0.9 ± 6.4%, while the overall measurement error by the measurement device according to the comparative example is -1.7 ± 6.3%. As described above, in the case of going down stairs with arm swing running, it is confirmed that the measurement error is reduced by performing the step measurement in consideration of the first piece count.

Note that, in the step measurement without consideration of the first piece count, the user's state may not be properly estimated, since the user's state is estimated only from the amplitude of the resultant acceleration waveform. For example, in the step measurement without consideration of the first piece count, substantially, the smaller the amplitude of the resultant acceleration waveform, the larger the time width of the partial waveform is estimated and the larger the lower-limit threshold value of the time width is set. Here, in the case of running down stairs, the amplitude of the resultant acceleration waveform is small, but the time width of the partial waveform is also small. Thus, in the step measurement without consideration of the first piece count, the lower-limit threshold value of the time width is set unreasonably large in the case of running down stairs. As a result, the partial waveform originating from running downstairs is regarded as a waveform originating from noise, resulting in counting omission, and the smaller number of steps than the true number of steps is measured.

Meanwhile, in the step measurement in consideration of the first piece count, the user's state is properly estimated, since the user's state is estimated from the amplitude of the resultant acceleration waveform and the first piece count. Accordingly, in the step measurement in consideration of the first piece count, the time width of the partial waveform is properly estimated and the lower-limit threshold value of the time width is properly set. Thus, in the step measurement in consideration of the first piece count, for example, the lower-limit threshold value of the time width is set properly small in the case of running down stairs. As a result, the partial waveform originating from running downstairs is properly regarded as a waveform originating from walking or running, and the number of steps close to the true number of steps is measured.

For example, in the above technique disclosed in Unexamined Japanese Patent Application Publication No. 2020-101849, depending on the user's state, a relationship between the amplitude of the acceleration waveform and the time width for one step in the acceleration waveform may not be in the above-described relationship. For example, when the user's state is a state of running down stairs, the amplitude of the acceleration waveform may be small and the time width for one step in the acceleration waveform may be short. In contrast to this, in the present embodiment, a reference range is determined based on the amplitude of the acceleration waveform and the first piece count, and one step is counted for the partial waveform when the time width of the partial waveform is discriminated as falling within the reference range. Accordingly, the present embodiment can properly discriminate whether the partial waveform is a waveform originating from walking or running, and can improve precision in step measurement.

Further, in the present embodiment, a state of a user wearing an acceleration sensor is estimated based on the amplitude of the acceleration waveform and the first piece count, and a reference range is determined based on the estimated state of the user. Accordingly, the present embodiment can set a proper reference range according to the user's state.

Further, in the present embodiment, the user's state is estimated to be a running downstairs state of running down stairs when the amplitude of the acceleration waveform is less than a first reference value and equal to or more than a second reference value smaller than the first reference value and the first piece count is equal to or more than a piece count threshold value. Further, the user's state is estimated to be a walking state when the amplitude of the acceleration waveform is less than the first reference value and the first piece count is less than the piece count threshold value or when the amplitude of the acceleration waveform is less than the second reference value. Accordingly, the present embodiment can properly discriminate the user's state as either the running downstairs state or the walking state.

Further, in the present embodiment, a lower-limit value of the reference range is determined as a first lower-limit value when the user's state is estimated to be the running downstairs state, and a lower-limit value of the reference range is determined as a second lower-limit value larger than the first lower-limit value when the user's state is estimated to be the walking state. Accordingly, the present embodiment can set a proper reference range according to the running downstairs state or the walking state.

Further, in the present embodiment, one step is counted for the partial waveform when the time width of the partial waveform is discriminated as falling within the reference range and the amplitude of the partial waveform is discriminated as being equal to or more than an amplitude threshold value. Accordingly, the present embodiment can further properly discriminate whether the partial waveform is a waveform originating from walking or running, and can further improve precision in step measurement.

Further, in the present embodiment, the amplitude threshold value is determined based on the amplitude of the acceleration waveform. Accordingly, the present embodiment can further properly discriminate whether the partial waveform is a waveform originating from walking or running, and can further improve precision in step measurement.

### Modified Example

While the embodiment has been described in the above, modifications and applications in various forms are possible. Any part of the configurations, functions, and operations described in the above embodiment may be employed. Further, besides the configurations, functions, and operations described above, additional configurations, functions, and operations may be employed. Further, the configurations, functions, and operations described in the above embodiment can be freely combined.

In the embodiment, an example has been described in which the user's state is discriminated as either the running downstairs state or the walking state based on both of the amplitude of the acceleration waveform and the first piece count when the amplitude of the acceleration waveform is less than the first reference value. The user's state may be discriminated as either the running downstairs state or the walking state based on only the first piece count when the amplitude of the acceleration waveform is less than the first reference value. In this case, the reference range determiner 104 estimates the user's state to be the running downstairs state of running down stairs when the amplitude of the acceleration waveform is less than the first reference value and the first piece count is equal to or more than the piece count threshold value. Further, the reference range determiner 104 estimates the user's state to be the walking state when the amplitude of the acceleration waveform is less than the first reference value and the first piece count is less than the piece count threshold value.

In the embodiment, an example has been described in which whether the partial waveform is a waveform originating from walking or running is discriminated from both of the time width of the partial waveform and the amplitude of the partial waveform. Whether the partial waveform is a waveform originating from walking or running may be discriminated from only the time width of the partial waveform.

In the embodiment, an example has been described in which the resultant acceleration data are calculated from three-axis acceleration data by calculating a square root of the sum of squares. The resultant acceleration data may be calculated from three-axis acceleration data by calculating a root mean square. In the embodiment, an example has been described in which the measurement device 100 acquires three-axis acceleration data and finds the resultant acceleration data from the three-axis acceleration data. The measurement device 100 may acquire two-axis acceleration data and find the resultant acceleration data from the two-axis acceleration data, or may acquire one-axis acceleration data and find the resultant acceleration data from the one-axis acceleration data. Note that, the measurement device 100 can find the resultant acceleration data by calculating a square root of the sum of squares or a root mean square.

In the embodiment, an example has been described in which t1 corresponding to the resultant acceleration that has changed from a negative value to a positive value among pieces of the time-series resultant acceleration is regarded as the first timing, and t2 corresponding to the resultant acceleration that has changed from a positive value to a negative value among pieces of the time-series resultant acceleration is regarded as the second timing. Timing t21 corresponding to the resultant acceleration immediately before changing from a negative value to a positive value among pieces of the time-series resultant acceleration may be regarded as the first timing, and timing t22 corresponding to the resultant acceleration that has changed from a positive value to a negative value among pieces of the time-series resultant acceleration may be regarded as the second timing. Further, timing t11 at which the resultant acceleration during ascending crosses zero may be regarded as the first timing, and timing 112 at which the resultant acceleration during descending crosses zero may be regarded as the second timing. Note that, t11 and t12 can be calculated by well-known interpolation processing. For example, an average value of t21 and t1 may be calculated as t11, and an average value of t22 and t2 may be calculated as t12.

In the embodiment, an example has been described in which the lower-limit threshold value being a lower-limit value of the reference range is a variable value, and the upper-limit threshold value being an upper-limit value of the reference range is a fixed value. Both of the lower-limit threshold value and the upper-limit threshold value may be variable values. In the embodiment, an example has been described in which the lower-limit threshold value for fast walking is a variable value according to the maximum acceleration. The lower-limit threshold value for fast walking may be a fixed value larger than Tc1 and smaller than Tc2.

In the embodiment, an example has been described in which a maximum value of the resultant acceleration in recent 1 second or a maximum difference value that is a difference between maximum and minimum values of the resultant acceleration in recent 1 second is regarded as the amplitude of the resultant acceleration waveform. A value of the DC component in the resultant acceleration in recent 1 second may be regarded as the amplitude of the resultant acceleration waveform.

In the embodiment, an example has been described in which the first piece count is used for discrimination between the running downstairs state and the walking state. The first piece count may be used for discrimination of other states. In the embodiment, an example has been described in which the user's state is estimated from the amplitude of the resultant acceleration waveform and the first piece count and the reference range for the time width of the partial waveform is determined from the user's state. The reference range may be determined from the amplitude of the resultant acceleration waveform and the first piece count without estimating the user's state.

In the above embodiment, the controller 11 functions as the units illustrated in FIG. 2 by the CPU executing the program stored in the ROM or the storage 12. However, in the present disclosure, the controller 11 may be dedicated hardware. The dedicated hardware is, for example, a single circuit, a complex circuit, a programmed processor, an application-specific integrated circuit (ASIC), a field-programmable gate array (FPGA), or a combination thereof. When the controller 11 is the dedicated hardware, each function of each unit may be achieved by individual piece of hardware, or the functions of the units may be collectively achieved by a single piece of hardware. Further, a part of the functions of the units may be achieved by dedicated hardware, and another part may be achieved by software or firmware. As described above, the controller 11 can achieve the above-described functions by hardware, software, firmware, or a combination thereof.

An operation program that defines the operation of the measurement device 100 according to the present disclosure can be applied to a computer such as an existing personal computer or an information terminal device, thereby causing the computer to function as the measurement device 100 according to the present disclosure. Further, such a program may be distributed by any method, for example, may be stored and distributed on a non-transitory computer-readable recording medium such as a compact disk ROM (CD-ROM), a digital versatile disk (DVD), a magneto optical disk (MO), or a memory card, or may be distributed over a communication network such as the Internet.

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions.

This application claims the benefit of Japanese Patent Application No. 2023-006827, filed January 19, 2023 which is hereby incorporated by reference wherein in its entirety.

The foregoing describes some example embodiments for explanatory purposes. Although the foregoing discussion has presented specific embodiments, persons skilled in the art will recognize that changes may be made in form and detail without departing from the broader spirit and scope of the invention. Accordingly, the specification and drawings are to be regarded in an illustrative rather than a restrictive sense. This detailed description, therefore, is not to be taken in a limiting sense, and the scope of the invention is defined only by the included claims, along with the full range of equivalents to which such claims are entitled.

## Claims

1. A measurement device (100) comprising:
an acceleration sensor (16);
a memory (12); and
one or more processors (11), wherein
the one or more processors (11) follow instructions saved in the memory (12) to execute following processing of:
acquiring time-series acceleration data output by the acceleration sensor (16);
specifying, based on the acquired time-series acceleration data, first timing at which acceleration goes above an acceleration reference value and second timing at which the acceleration goes below the acceleration reference value;
determining a reference range for a time width from the first timing to the second timing based on amplitude of an acceleration waveform corresponding to the time-series acceleration data and a first piece count of at least either the first timing or the second timing within predetermined time;
determining whether the time width falls within the determined reference range; and
counting one step for a partial waveform from the first timing to the second timing in the acceleration waveform when the time width is determined as falling within the reference range.

2. The measurement device (100) according to claim 1, wherein determining the reference range includes determining a state of a user wearing the acceleration sensor (16) based on amplitude of the acceleration waveform and the first piece count, and determining the reference range based on the determined state of the user.

3. The measurement device (100) according to claim 2, wherein determining the reference range includes determining a state of the user to be a running downstairs state of running down stairs when amplitude of the acceleration waveform is less than a first reference value and the first piece count is equal to or more than a piece count threshold value, and determining a state of the user to be a walking state when amplitude of the acceleration waveform is less than the first reference value and the first piece count is less than the piece count threshold value.

4. The measurement device (100) according to claim 2, wherein determining the reference range includes determining a state of the user to be a running downstairs state of running down stairs when amplitude of the acceleration waveform is less than a first reference value and equal to or more than a second reference value smaller than the first reference value and the first piece count is equal to or more than a piece count threshold value, and determining a state of the user to be a walking state when amplitude of the acceleration waveform is less than the first reference value and the first piece count is less than the piece count threshold value or when amplitude of the acceleration waveform is less than the second reference value.

5. The measurement device (100) according to claim 3, wherein determining the reference range includes determining a lower-limit value of the reference range as a first lower-limit value when a state of the user is determined to be the running downstairs state, and determining a lower-limit value of the reference range as a second lower-limit value larger than the first lower-limit value when a state of the user is determined to be the walking state.

6. The measurement device (100) according to claim 4, wherein determining the reference range includes determining a lower-limit value of the reference range as a first lower-limit value when a state of the user is determined to be the running downstairs state, and determining a lower-limit value of the reference range as a second lower-limit value larger than the first lower-limit value when a state of the user is determined to be the walking state.

7. The measurement device (100) according to any one of claims 1 to 4, wherein the one or more processors (11) further
determine whether amplitude of the partial waveform is equal to or more than an amplitude threshold value, and,
in the counting, count one step for the partial waveform when the time width is determined as falling within the reference range and amplitude of the partial waveform is determined as being equal to or more than the amplitude threshold value.

8. The measurement device (100) according to claim 7, wherein the one or more processors (11) further determine the amplitude threshold value based on amplitude of the acceleration waveform.

9. A measurement method executed by a measurement device (100) including an acceleration sensor (16), comprising:
acquiring time-series acceleration data output by the acceleration sensor (16);
specifying, based on the acquired time-series acceleration data, first timing at which acceleration goes above an acceleration reference value and second timing at which the acceleration goes below the acceleration reference value;
determining a reference range for a time width from the first timing to the second timing based on amplitude of an acceleration waveform corresponding to the time-series acceleration data and a first piece count of at least either the first timing or the second timing within predetermined time;
determining whether the time width falls within the determined reference range; and
counting one step for a partial waveform from the first timing to the second timing in the acceleration waveform when the time width is determined as falling within the reference range.

10. The measurement method according to claim 9, wherein determining the reference range includes determining a state of a user wearing the acceleration sensor (16) based on amplitude of the acceleration waveform and the first piece count, and determining the reference range based on the determined state of the user.

11. The measurement method according to claim 10, wherein determining the reference range includes determining a state of the user to be a running downstairs state of running down stairs when amplitude of the acceleration waveform is less than a first reference value and the first piece count is equal to or more than a piece count threshold value, and determining a state of the user to be a walking state when amplitude of the acceleration waveform is less than the first reference value and the first piece count is less than the piece count threshold value.

12. The measurement method according to claim 10, wherein determining the reference range includes determining a state of the user to be a running downstairs state of running down stairs when amplitude of the acceleration waveform is less than a first reference value and equal to or more than a second reference value smaller than the first reference value and the first piece count is equal to or more than a piece count threshold value, and determining a state of the user to be a walking state when amplitude of the acceleration waveform is less than the first reference value and the first piece count is less than the piece count threshold value or when amplitude of the acceleration waveform is less than the second reference value.

13. The measurement method according to claim 11, wherein determining the reference range includes determining a lower-limit value of the reference range as a first lower-limit value when a state of the user is determined to be the running downstairs state, and determining a lower-limit value of the reference range as a second lower-limit value larger than the first lower-limit value when a state of the user is determined to be the walking state.

14. The measurement method according to any one of claims 9 to 12, further comprising:
determining whether amplitude of the partial waveform is equal to or more than an amplitude threshold value; and
counting one step for the partial waveform when the time width is determined as falling within the reference range and amplitude of the partial waveform is determined as being equal to or more than the amplitude threshold value.

15. A program executable by one or more processors (11) in a measurement device (100),
wherein the measurement device (100) includes an acceleration sensor (16), and
the program causing the one or more processors (11) to perform:
acquiring time-series acceleration data output by the acceleration sensor (16);
specifying, based on the acquired time-series acceleration data, first timing at which acceleration goes above an acceleration reference value and second timing at which the acceleration goes below the acceleration reference value;
determining a reference range for a time width from the first timing to the second timing based on amplitude of an acceleration waveform corresponding to the time-series acceleration data and a first piece count of at least either the first timing or the second timing within predetermined time;
determining whether the time width falls within the determined reference range; and
counting one step for a partial waveform from the first timing to the second timing in the acceleration waveform when the time width is determined as falling within the reference range.

16. The program according to claim 15, wherein determining the reference range includes determining a state of a user wearing the acceleration sensor (16) based on amplitude of the acceleration waveform and the first piece count, and determining the reference range based on the determined state of the user.

17. The program according to claim 16, wherein determining the reference range includes determining a state of the user to be a running downstairs state of running down stairs when amplitude of the acceleration waveform is less than a first reference value and the first piece count is equal to or more than a piece count threshold value, and determining a state of the user to be a walking state when amplitude of the acceleration waveform is less than the first reference value and the first piece count is less than the piece count threshold value.

18. The program according to claim 16, wherein determining the reference range includes determining a state of the user to be a running downstairs state of running down stairs when amplitude of the acceleration waveform is less than a first reference value and equal to or more than a second reference value smaller than the first reference value and the first piece count is equal to or more than a piece count threshold value, and determining a state of the user to be a walking state when amplitude of the acceleration waveform is less than the first reference value and the first piece count is less than the piece count threshold value or when amplitude of the acceleration waveform is less than the second reference value.

19. The program according to claim 17, wherein determining the reference range includes determining a lower-limit value of the reference range as a first lower-limit value when a state of the user is determined to be the running downstairs state, and determining a lower-limit value of the reference range as a second lower-limit value larger than the first lower-limit value when a state of the user is determined to be the walking state.

20. The program according to any one of claims 15 to 18, the program further causing the one or more processors (11) to perform:
determining whether amplitude of the partial waveform is equal to or more than an amplitude threshold value; and
counting one step for the partial waveform when the time width is determined as falling within the reference range and amplitude of the partial waveform is determined as being equal to or more than the amplitude threshold value.
